Europäisches Patentamt

European Patent Office

Office européen des brevets

(19)

(11) Numéro de publication : **0 101 356**
**B1**

(12) **FASCICULE DE BREVET EUROPÉEN**

(45) Date de publication du fascicule du brevet :
15.05.85

(21) Numéro de dépôt : **83401507.5**

(22) Date de dépôt : **22.07.83**

(51) Int. Cl.⁴ : **C 07 C149/06**, C 07 C148/00

(54) **Procédé de synthèse de mercaptans à partir d'oléfines et d'hydrogène sulfuré, par catalyse hétérogène.**

(30) Priorité : 05.08.82 FR 8213689

(43) Date de publication de la demande :
22.02.84 Bulletin 84/08

(45) Mention de la délivrance du brevet :
15.05.85 Bulletin 85/20

(84) Etats contractants désignés :
DE GB NL

(56) Documents cités :
CHEMICAL ABSTRACTS, vol. 95, no. 11, 14 septembre 1981, page 587, no. 97053s, Columbus, Ohio, USA

(73) Titulaire : **SOCIETE NATIONALE ELF AQUITAINE (PRODUCTION)**
**Tour Aquitaine**
**F-92400 Courbevoie (FR)**

(72) Inventeur : **Arretz, Emmanuel**
**2 Rue de Cagnes**
**F-64000 Pau (FR)**
Inventeur : **Mirassou, Alfred**
**Poey de Lescar**
**F-64230 Lescar (FR)**
Inventeur : **Landoussy, Claude**
**12 rue du Pourtalet**
**F-64000 Pau (FR)**
Inventeur : **Auge, Patrick**
**4, rue de Nogaro**
**F-64000 Pau (FR)**

(74) Mandataire : **Kohn, Armand**
**5 Avenue Foch**
**F-92380 Garches (FR)**

Jouve, 18, rue St-Denis, 75001 Paris, France

## Description

L'invention concerne un procédé pour la synthèse d'un mercaptan à partir d'une oléfine, par l'action de l'hydrogène sulfuré sur celle-ci, en présence d'un catalyseur insoluble. L'invention vise plus spécialement un procédé, dans lequel le catalyseur est constitué par un échangeur de cations, à savoir une résine échangeuse de cations. Ce nouveau procédé convient particulièrement à la production de mercaptans tertiaires, notamment à celle de tertiododécyl-mercaptan à partir de tétrapropylène ou de tri-isobutylène.

L'utilité industrielle de différents mercaptans a fait que des procédés de production en ont été très étudiés depuis une trentaine d'années. Aussi, la littérature technique comporte-t-elle d'abondants documents sur cette question. Les procédés, qui étaient d'abord employés, étaient basés sur la catalyse en milieu homogène par des composés acides, notamment des acides de Lewis, plus particulièrement chlorure d'aluminium et fluorure de bore. Pour des raisons évidentes, on a cherché à remplacer ces catalyseurs solubles par des masses catalytiques minérales, insolubles, surtout à base de silice et d'alumine. Ces derniers exigeaient des températures élevées et conduisaient à des rendements laissant à désirer. Un progrès a été réalisé par l'utilisation de silico-aluminates sous une forme spéciale, celle de zéolithes. Un tel procédé est décrit dans la publication de brevet français nº 2 391 197. Cependant, là encore on est obligé de travailler à des températures relativement élevées, de l'ordre de 90 °C au moins pour avoir des productivités industrielles viables. L'activité du catalyseur baisse sensiblement, déjà après une centaine d'heures d'utilisation, en présence de l'eau contenue dans les réactifs, ce qui oblige à de fréquentes régénérations du catalyseur et un séchage rigoureux des réactifs.

Il semble qu'auparavant des chercheurs aient essayé d'utiliser des échangeurs organiques de cations : ainsi le brevet tchèque nº 185 469 préconise-t-il l'emploi des résines échangeuses à base de copolymères styrène-divinylbenzène ou phénol-formaldéhyde. Mais il apparaît que, dans les conditions opératoires appliquées, de telles résines catalysent surtout la réaction du mercaptan sur l'oléfine pour donner un thioéther, et peu celle de l'$H_2S$ sur l'oléfine, donnant du mercaptan. C'est sans doute la raison pour laquelle les rendements en thiol, selon le brevet tchèque (ex. 3 et 4), sont très faibles, par contre, ceux de thioéther atteignent 80 %.

Cette production exigerait d'ailleurs — d'après les auteurs — une certaine humidité non négligeable et une température d'au moins 80 °C. En définitive, bien que le document en question parle tant de la synthèse des thioéthers que de celle des thiols, cette dernière paraissait industriellement impossible en présence des résines échangeuses d'ions. D'autre part, malgré l'affirmation, dans la plupart des brevets antérieurs, que la préparation des mercaptans peut s'effectuer dans un large domaine de températures, notamment entre 20° et 200 °C ou 50° à 150 °C, il n'a pas été possible d'obtenir des résultats industriels, par les procédés connus, à des températures inférieures à 90 °C.

Contrairement à l'ensemble de l'art antérieur, le procédé suivant l'invention apporte le perfectionnement imprévu de permettre l'obtention, avec d'excellents résultats, de mercaptans à des températures assez basses, notamment inférieures à 80 °C avec, comme catalyseur, une résine échangeuse de cations. Du fait même de l'opération à températures très modérées, le catalyseur reste actif pendant très longtemps et ne conduit pas à la baisse de rendement après une centaine d'heures, comme c'est le cas pour d'autres catalyseurs. Il est également surprenant, devant les faits exposés dans le brevet tchèque sus-indiqué, que le procédé suivant l'invention, utilisant une résine échangeuse de cations, permette l'obtention de thiols très purs, avec de bons rendements et avec une sélectivité pratiquement de 100 %.

L'invention résulte de la constatation, contraire à l'art antérieur, que des résines échangeuses de cations peuvent bien catalyser l'addition de l'$H_2S$ à des oléfines pour donner des mercaptans avec d'excellents rendements et pureté, à condition que la résine soit suffisamment sèche et que la température soit réglée entre des limites rigoureuses de 45° à 75 °C, ou mieux entre 50° et 70 °C.

Ainsi donc, le nouveau procédé suivant l'invention, consiste-t-il à mettre en contact de l'hydrogène sulfuré avec une ou plusieurs oléfines en présence d'une résine échangeuse de cations exempte au maximum d'humidité, à une température de 45 à 75 °C, de préférence sous une pression relative de 1 à 50 bars.

L'opération peut être menée suivant tout mode opératoire connu en soi ; elle peut être réalisée en discontinu par injection de l'oléfine et de $H_2S$ dans un réacteur renfermant une charge de résine échangeuse d'ions, chauffé à la température voulue, pendant le temps nécessaire, après quoi, le liquide formé est séparé à la manière connue. On peut également travailler en continu, dans un réacteur tubulaire de longueur suffisante, chargé de résine échangeuse, et alimenté à une de ses extrémités par un courant d'oléfine et de $H_2S$, le produit de la réaction étant recueilli à l'autre bout du tube, pour être purifié.

En tant que catalyseur, dans le procédé suivant l'invention, conviennent les différents polymères et copolymères à fonctions acides, connus dans l'art comme échangeurs de cations. En particulier, on peut employer des résines à base de polystyrène sulfoné, réticulées, en particulier avec du divinylbenzène, des résines acryliques ou phénylacryliques, à groupes carboxyliques libres, des résines type phénol formaldéhyde dérivé des acides phénol sulfoniques, des échangeurs ligno sulfoniques, etc. Des résines de ce genre se trouvent dans le commerce sous différentes dénominations, en particulier Aliassion, Cecacit, Wofatites et Levatites, Imac, Ionac, Amberlites, Liquorex, Zeorex, Zeocarb, Dowex, etc. Conviennent tout particulièrement les copolymères du styrène sulfoné avec du divinyl benzène, par exemple ceux que l'on trouve dans le commerce sous les dénominations Amberlyst, Lewatit ou Dowex ; d'autre part, peuvent être employés avantageusement les copolymères de tétrafluoroéthylène avec de l'acide perfluorosulfonique, en particulier l'acide per-fluoro-3,6-dioxa-4-méthyl-7-octène sulfonique connu sous la marque de fabrique Nafion.

Une des conditions essentielles de l'invention étant la siccité maximum de la résine employée comme catalyseur, on doit veiller à ce que cette résine ne contienne pas plus de 0,5 % d'eau déterminable par séchage à 80° pendant 6 heures, et de préférence 0,01 à 0,2 %.

Bien qu'il soit utile, pour des raisons chimiques, d'effectuer la synthèse sous une pression élevée, par exemple sous 50 bars, l'invention permet de travailler sous des pressions modérées, notamment sous 10 à 16 bars, c'est-à-dire dans des conditions technologiquement aisées, tout en obtenant d'excellents résultats. Il est néanmoins possible d'opérer à toute pression relative entre 1 et 50 bars, la marge de 10 à 16 bars étant cependant industriellement la plus favorable.

Comme dans les procédés connus, il convient d'employer un certain excès suffisant d'hydrogène sulfuré : les rapports molaires pratiques $H_2S$/oléfine étant de 1,2 à 10, et de préférence 2 à 5.

Les exemples qui suivent, non limitatifs, illustrent l'invention.

Exemple 1

Dans un réacteur tubulaire d'une longueur de 135 mm et d'un diamètre intérieur de 20 mm, soit d'une capacité de 420 ml, on place 200 ml de résine sulfonée de copolymère styrène-divinylbenzène (Amberlyst 15) sèche. Sous une pression de 10 bars, on introduit, en continu, du tétrapropylène liquide, à raison de 121 g/h, dans ce réacteur ; en même temps, on y injecte 73 g/h de $H_2S$ gazeux. Le milieu réactionnel est maintenu à la température de 60 °C ± 2 °C. Les réactifs sont mélangés intimement avant leur passage dans le réacteur. Le liquide s'écoulant en continu du réacteur, est recueilli et l'hydrogène sulfuré restant est dégazé.

Dans le liquide obtenu, on retrouve 8 % de tétrapropylène non transformé, le reste étant du tertio dodécyl mercaptan : celui-ci est donc obtenu avec un taux de conversion de l'oléfine de 92 %, avec une sélectivité de 100 %.

Une opération similaire est effectuée, avec cette seule différence, que le catalyseur contient 1,6 % d'humidité, la conversion du tétrapropylène n'est plus alors que de 80 %.

Exemple 2

Une préparation similaire à celle de l'exemple 1 est effectuée dans le même réacteur, mais avec des débits de réactifs deux fois plus lents : on introduit ainsi 60 g/h de tétrapropylène et 36,7 g/h d'hydrogène sulfuré, sous 10 bars. La conversion du tétrapropylène est alors de 96 % et la sélectivité du tertio dodécyl mercaptan obtenu de 100 %. Comme la production du mercaptan représente encore 69,25 g/h par litre de l'espace catalytique, les conditions opératoires sont tout à fait acceptables industriellement et donnent un très bon rendement avec une pureté parfaite du produit. Comme aucune impureté ne se forme, le tétrapropylène non transformé est aisément recyclable.

Dans les mêmes conditions opératoires, mais avec de la résine Amberlyst 15 humide, la conversion du tétrapropylène chute à 85 %.

Exemple 3

Dans le même réacteur que précédemment, on opère avec 100 g de résine perfluorosulfonique acide, sèche. On introduit, sous 10 bars, 60 g/h de tétrapropylène et 36,7 g/h d'hydrogène sulfuré. En sortie du réacteur, on obtient une conversion du tétrapropylène de 90 % avec une sélectivité en tertiododécylmercaptan pratiquement de 100 %.

Exemple 4

En opérant avec 120 g de résine Amberlyst 15, on introduit dans le réacteur, sous une pression de 10 bars, 40 g/h d'isobutène et 73 g/h d'hydrogène sulfuré. La température de réaction est de 70 °C. On obtient 63 g/h de tertiobutylmercaptan, soit une conversion de 98 % en ce produit.

Exemple 5

On a opéré comme dans l'exemple 1, avec de la résine sèche Amberlyst 15 et avec des débits de 121 g/h de tétrapropylène et 73 g/h d'hydrogène sulfuré, à une température de 60 °C. L'essai a été poursuivi pendant 700 heures sans modification de la conversion du tétrapropylène, qui s'est maintenue à 91-92 %.

**Revendications**

1. Procédé pour la synthèse d'un mercaptan à partir d'une oléfine, par l'action de l'hydrogène sulfuré sur celle-ci, en présence d'un catalyseur constitué par une résine échangeuse de cations, caractérisé en ce que cette résine est sèche et que la réaction est effectuée à une température comprise entre 45° et 75 °C.

2. Procédé suivant la revendication 1, caractérisé en ce que la température est maintenue entre 50° et 70 °C.

3. Procédé suivant la revendication 1 ou 2, dans lequel la résine échangeuse de cations est un copolymère de styrène sulfoné avec du divinylbenzène.

4. Procédé suivant la revendication 1 ou 2, caractérisé en ce que la résine échangeuse de cations est constituée par un copolymère de tétrafluoroéthylène avec de l'acide perfluorosulfonique.

5. Procédé suivant une des revendications précédentes dans lequel le milieu réactionnel est maintenu sous une pression de 1 à 50 bars et plus spécialement sous 10 à 16 bars.

6. Procédé suivant une des revendications précédentes, caractérisé en ce que la résine échangeuse de cations ne renferme pas plus de 0,5 % d'eau, cette humidité étant déterminée par le séchage à 80 °C pendant 6 heures.

7. Procédé suivant la revendication 6, caractérisé en ce que la teneur en eau du milieu réactionnel est de 0,01 à 0,2 %.

8. Application du procédé, suivant une des revendications 1 à 7, à la production de mercaptans tertiaires, en particulier du tertio-dodécylmercaptan.

## Claims

1. A process for the synthesis of a mercaptan from an olefin by the action of hydrogen sulphide thereon in the presence of a catalyst comprising a cation exchange resin, characterised in that this resin is dry and that the reaction is effected at a temperature ranging between 45° and 75 °C.

2. A process according to claim 1, characterised in that the temperature is maintained between 50° and 70 °C.

3. Process according to claim 1 or 2, in which the cation exchange resin is a copolymer of sulphonated styrene with divinylbenzene.

4. A process according to claim 1 or 2, characterised in that the cation exchange resin comprises a copolymer of tetrafluoroethylene with perfluorosulphonic acid.

5. A process according to any of the preceding claims, in which the reaction medium is maintained under a pressure of 1 to 50 bars and more especially under 10 to 16 bars.

6. A process according to any of the preceding claims, characterised in that the cation exchange resin does not contain more than 0.5 % of water, this moisture content being determined by drying at 80 °C for 6 hours.

7. A process according to claim 6, characterised in that the water content of the reaction medium is from 0.01 to 0.2 %.

8. Application of the process according to any of claims 1 to 7 to the production of tertiary mercaptans, in particular tertio-dodecyl-mercaptan.

## Patentansprüche

1. Verfahren zur Synthese eines Mercaptans ausgehend von einem Olefin, durch Einwirken von Schewefelwasserstoff auf dieses, in Anwesenheit eines Katalysators, der aus einem Kationenaustauscherharz besteht, dadurch gekennzeichnet, daß dieses Harz trocken ist und daß die Reaktion bei einer Temperatur von 45 bis 75 °C durchgeführt wird.

2. Verfahren nach Anspruch 1, dadurch gekennzeichnet, daß die Temperatur bei 50 bis 70 °C gehalten wird.

3. Verfahren nach Anspruch 1 oder 2, bei dem das Kationenaustauscherharz ein Copolymeres von sulfoniertem Styrol mit Divinylbenzol ist.

4. Verfahren nach Anspruch 1 oder 2, dadurch gekennzeichnet, daß das Kationenaustauscherharz aus einem Copolymeren von Tetrafluorethylen mit Perfluorsulfonsäure besteht.

5. Verfahren nach einem der vorhergehenden Ansprüche, bei dem das Reaktionsmedium unter einem Druck von 1 bis 50 bar und insbesondere unter 10 bis 16 bar gehalten wird.

6. Verfahren nach einem der vorhergehenden Ansprüche, dadurch gekennzeichnet, daß das Kationenaustauscherharz nicht mehr als 0,5 % Wasser einschließt, wobei diese Feuchtigkeit durch 6 h Trocknen bei 80 °C bewirkt wird.

7. Verfahren nach Anspruch 6, dadurch gekennzeichnet, daß der Wassergehalt des Reaktionsmediums 0,01 bis 0,2 % beträgt.

8. Verwendung des Verfahrens gemäß einem der Ansprüche 1 bis 7, zur Herstellung von tertiären Mercaptanen, insbesondere von tert. Dodecylmercaptan.